# EUROPEAN PATENT APPLICATION

(11) **EP 3 447 031 A1**
(43) Date of publication of application: **27.02.2019**
(21) Application number: 17880847.3
(22) Date of filing: 07.12.2017
(51) Int. Cl.: C02F 9/02, C02F 9/08, C07C 273/16, C02F 1/20, C02F 1/28, C02F 1/42, C02F 1/44, C02F 1/469

(54) **METHOD AND DEVICE FOR PREPARING UREA SOLUTION FOR VEHICLE**

(30) Priority: 14.12.2016 CN 201611158489
(71) Applicant: Zhuhai Gree Intelligent Equipment Co., Ltd, Zhuhai, Guangdong 519070 (CN); Gree Electric Appliances, Inc. of Zhuhai, Zhuhai, Guangdong 519070 (CN)
(72) Inventor: ZHANG, Xiufeng, Zhuhai Guangdong 519070 (CN); GONG, Ke, Zhuhai Guangdong 519070 (CN); XU, Lihai, Zhuhai Guangdong 519070 (CN); ZHANG, Liang, Zhuhai Guangdong 519070 (CN); ZHAI, Xinchun, Zhuhai Guangdong 519070 (CN); WANG, Zhong, Zhuhai Guangdong 519070 (CN); ZENG, Wei, Zhuhai Guangdong 519070 (CN)
(74) Representative: Zacco GmbH
(86) International application number: PCT/CN2017/115067
(87) International publication number: WO 2018/108027

(57) **Abstract**

A preparation method for vehicle urea solution, comprising: a pure water preparation device (11) is controlled to perform water treatment on raw water, and pure water is obtained; the pure water is heated to obtain heated water; the heated water and urea particles are stirred in a stirring tank (14), the urea solution is obtained. A preparation device for vehicle urea solution, comprising: the pure water preparation device (11), a heater (13) and the stirring tank (14).

## Description

### Technical Field

The embodiments of the disclosure relate to the field of urea solution preparation, in particular to a preparation method and device for vehicle urea solution.

### Background

The vehicle urea solution at present is obtained in an industrialized production mode, production equipment is long in working procedure, large in land occupation, and low in efficiency, and multiple professionals are needed simultaneously for operation and maintenance, in addition, in order to better store the urea solution, dangerous chemical medicines, such as strong acid and strong base, are needed for using, and the security is worse.

In allusion to a problem that the preparation efficiency of the above preparation method for the urea solution is low, there hasn't any effective solution yet till now.

### Summary

The disclosure provides a preparation method and device for vehicle urea solution, and aims to at least solve a technical problem that the preparation efficiency of the preparation method for the urea solution is low.

According to one aspect of the embodiments of the disclosure, a preparation method for vehicle urea solution is provided, including: a pure water preparation device is controlled to perform water treatment on raw water, and pure water is obtained; the pure water is heated to obtain heated water; the heated water and urea particles are stirred in a stirring tank, the urea solution is obtained.

In an optional embodiment of the disclosure, the step of controlling the pure water preparation device to perform water treatment on raw water includes the following steps: the raw water is pre-treated through a pre-treatment unit, and first-level treated water is obtained; the first-level treated water is filtered by a reverse osmosis (RO) treatment unit, and second-level treated water is obtained; the second-level treated water is filtered by using an ion exchange technology, and the pure water is obtained.

In another optional embodiment of the disclosure, the step of pre-treating the raw water through the pre-treatment unit includes the following step: at least one filter is used for filtering sand grains, solid particles, colloid and microorganisms in the raw water, and the first-level treated water is obtained, wherein the pre-treatment unit includes at least one filter.

In another optional embodiment of the disclosure, the step of pre-treating the raw water through the pre-treatment unit includes the following steps: a pre-filter, a winding candle filter, a granular active carbon filter, and an ultrafiltration membrane element are successively used for filtering the raw water, wherein the pre-treatment unit includes the pre-filter, the winding candle filter, the granular active carbon filter, and the ultrafiltration membrane element.

In another optional embodiment of the disclosure, the step of filtering the first-level treated water by an RO treatment unit includes the following steps: ions in the first-level treated water are removed by a two-level RO treatment unit, and the second-level treated water is obtained.

In another optional embodiment of the disclosure, the step of filtering the second-level treated water by using an ion exchange technology includes the following step: an electrodeionization (EDI) system is used for filtering trace ions in the second-level treated water.

In another optional embodiment of the disclosure, after the EDI system is used for filtering trace ions in the second-level treated water, the content of carbon dioxide in the water in which the trace ions are filtered is detected; in the case that the content of the carbon dioxide exceeds a preset content, a degasser is used for removing the carbon dioxide in the water in which the trace ions are filtered.

In another optional embodiment of the disclosure, after the EDI system is used for filtering trace ions in the second-level treated water, the method further includes the following step: a resin component is used for purifying water in which the trace ions are filtered.

In another optional embodiment of the disclosure, the step of heating the pure water includes the following steps: while the pure water is flowed into a heat-insulating water tank, a compressor is started, and under the drive of the compressor, heat is transmitted to a condenser, the heat is released to the water in the heat-insulating water tank, so that the pure water is heated.

In another optional embodiment of the disclosure, the step of starting the compressor includes the following steps: the temperature of the heat-insulating water tank is detected, in the case that the temperature of the heat-insulating water tank is lower than a first threshold value, the compressor is started; after the compressor is started, the method further includes: in the case that the detected temperature of the heat-insulating water tank is higher than a second threshold valve, the compressor is controlled to be stopped.

In another optional embodiment of the disclosure, the step of stirring the heated water and urea particles in a stirring tank includes the following steps: while a volume of the heated water entering the stirring tank reaches a first preset quantity, the stirring is started, and in the case that a preset condition is met, the urea particles are absorbed into the stirring tank.

In another optional embodiment of the disclosure, the step of circularly stirring the heated water and urea particles in the stirring tank includes the following steps: a circulating pump is used for pumping out solution in the stirring tank from a circulating stirring output pipe, and entering from a circulating stirring input pipe, so that the circulating stirring is performed until the concentration of the urea solution in the stirring tank reaches a preset concentration.

In another optional embodiment of the disclosure, the method further includes the following step: after the pure water is obtained, the pure water is stored in an ultrapure water tank.

According to one aspect of the embodiments of the disclosure, a preparation device for vehicle urea solution is provided. The preparation device includes a pure water preparation device configured to perform water treatment on raw water to obtain pure water; a heater configured to heat the pure water to obtain heated water; and a stirring tank configured to stir the heated water and urea particles to obtain urea solution.

In another optional embodiment of the disclosure, the pure water preparation device includes a pre-treatment unit, configured to perform the pre-treatment on raw water to obtain first-level treated water; an RO treatment unit, configured to filter the first-level treated water to obtain second-level treated water; and a pure water manufacturing device configured to filter the second-level treated water by using an ion exchange technology to obtain the pure water.

In another optional embodiment of the disclosure, the pre-treatment unit includes at least one filter configured to filter sand grains, solid particles, colloid and microorganisms in the raw water to obtain the first-level treated water.

In another optional embodiment of the disclosure, the pre-treatment unit includes a pre-filter, a winding candle filter, a granular active carbon filter, and an ultrafiltration membrane element.

In another optional embodiment of the disclosure, the pure water preparation device further includes a detector configured to detect content of carbon dioxide in the water in which the trace ions are filtered; and a degasser, configured to, in the case that the content of the carbon dioxide exceeds a preset content, remove the carbon dioxide in the water in which the trace ions are filtered.

In another optional embodiment of the disclosure, the pure water preparation device further includes a resin component, configured to, after the EDI system is used for filtering trace ions in the second-level treated water, purify the water in which the trace ions are filtered.

In another optional embodiment of the disclosure, the stirring tank is provided with a circulating pump, the circulating pump is configured to pump out the urea solution in the stirring tank from a circulating stirring output pipe, and enter from a circulating stirring input pipe, so that the circulating stirring is performed, wherein a connecting port of the circulating stirring input pipe and the stirring tank is positioned between the bottom of the stirring tank and the middle of the stirring tank.

In another optional embodiment of the disclosure, the preparation device further includes an ultrapure water tank, the ultrapure water tank is configured to store the pure water.

In the embodiments of the disclosure, the preparation device for the vehicle urea solution is a complete machine device, and through the pure water preparation device, the pure water tank, the heater, the stirring tank and a storage box, devices of pure water preparation, pure water heating, solution stirring and the like are integrated, each device is synergistically operated, so that the preparation for the urea solution may be rapidly completed. In addition, the preparation device in the above embodiments is capable of breaking the original industrialized production mode, realizing the miniaturization and automation of the devices. Through the above embodiments, the problem in the related art that the low preparation efficiency of the urea solution is solved, and the preparation efficiency of the urea solution is improved.

### Brief Description of the Drawings

The accompanying drawings are described here to provide a further understanding to the disclosure, and form a part of the disclosure. The schematic embodiments and description of the disclosure are adopted to explain the disclosure, and do not form improper limits to the disclosure. In the drawings:
FIG. 1 is a schematic diagram of a preparation device for vehicle urea solution according to an embodiment of the disclosure;
FIG. 2 is a schematic diagram of another preparation device for vehicle urea solution according to an embodiment of the disclosure;
FIG. 3 is a schematic diagram of a pre-treatment unit of a pure water preparation device according to an embodiment of the disclosure;
FIG. 4 is a schematic diagram of an RO treatment unit according to an embodiment of the disclosure;
FIG. 5 is a schematic diagram of an EDI system according to an embodiment of the disclosure;
FIG. 6 is a schematic diagram of another preparation device for vehicle urea solution according to an embodiment of the disclosure;
FIG. 7 is a flow diagram of a preparation method for vehicle urea solution according to an embodiment of the disclosure;
FIG. 8 is an optional heating schematic diagram according to an embodiment of the disclosure;
FIG. 9 is an optional stirring schematic diagram according to an embodiment of the disclosure.

### Detailed Description of the Embodiments

In order to make those skilled in the art better understand the solutions of the disclosure, the technical solutions in the embodiments of the disclosure will be clearly and completely described herein below with reference to the drawings in the embodiments of the disclosure. It is apparent that the described embodiments are only a part of the embodiments of the disclosure instead of all. On the basis of the embodiments of the disclosure, all other embodiments obtained on the premise of no creative work of those of ordinary skill in the art shall fall within the scope of protection of the disclosure.

It is to be noted that the specification and claims of the disclosure and terms "first", "second" and the like in the drawings are intended to distinguish similar objects, and do not need to describe a specific sequence or a precedence order. It is to be understood that data used in such a way may be exchanged under appropriate conditions, in order that the embodiments of the disclosure described here can be implemented in a sequence except sequences graphically shown or described here. In addition, terms "comprise", "include" and variations thereof are intended to cover non-exclusive inclusions. For example, processes, methods, systems, products or devices containing a series of steps or units do not need to clearly show those steps or units, and may include other inherent steps or units of these processes, methods, products or devices, which are not clearly shown.

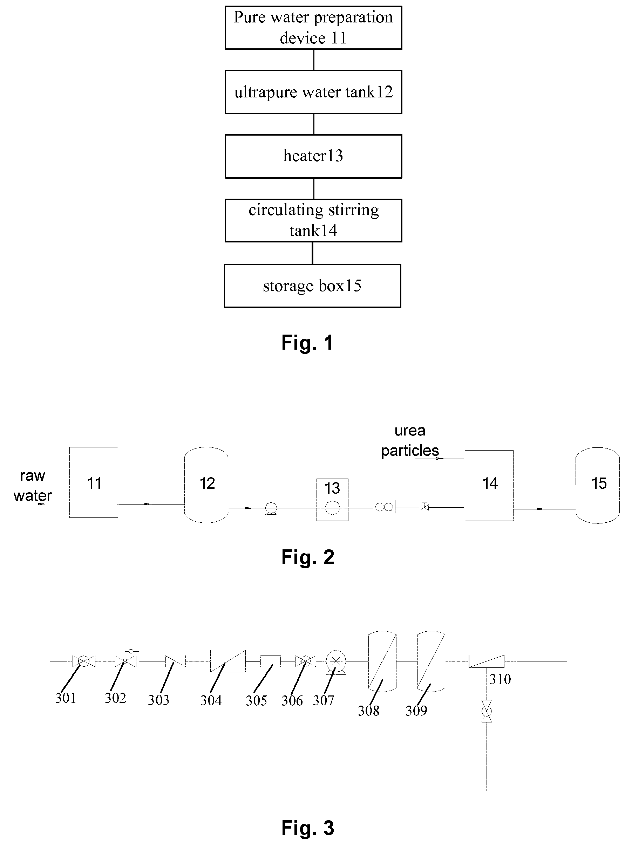

According to one aspect of the disclosure embodiment, a preparation device for vehicle urea solution is provided. The device, as shown in FIG. 1, includes: a pure water preparation device 11, configured to perform water treatment on raw water to obtain pure water; a heater 13, configured to heat the pure water to obtain heated water; and a stirring tank 14, configured to stir the heated water and urea particles to obtain urea solution. The pure water in the embodiment of the disclosure may be ultrapure water.

The preparation device for the vehicle urea solution in the above embodiment is a complete machine device, through the pure water preparation device, the heater, and the stirring tank, devices of pure water preparation, pure water heating, solution stirring and the like are integrated, each device is synergistically operated, so that the preparation for the urea solution may be rapidly completed. In addition, the preparation device in the above embodiment is capable of breaking the original industrialized production mode, realizing the miniaturization and automation of the devices. Through the above embodiment, the problem in the related art that the low preparation efficiency of the urea solution is solved, and the preparation efficiency of the urea solution is improved.

As shown in FIG. 2, a pure water preparation device 11 of the preparation device, a pure water tank 12 (such as an ultrapure water tank), a heater 13 and a circulating stirring tank 14 (such as the above stirring tank) and a storage box 15 are successively connected as a complete machine. While the preparation device is used, in an initial starting process, the pure water preparation device treats raw water to generate pure water which is flowed into the pure water tank, the pure water in the pure water tank is heated by the heater and is flowed into the circulating stirring tank for uniformly stirring urea particles and heated water, and solution is pumped out and flowed into the storage box. In a stable operation process, each module may be independently operated.

The pure water preparation device in the above embodiment may include a pre-treatment unit configured to perform the pre-treatment on raw water to obtain first-level treated water; an RO treatment unit configured to filter the first-level treated water to obtain second-level treated water; and a pure water manufacturing device configured to filter the second-level treated water by using an ion exchange technology to obtain the pure water.

Through three parts of a pre-treatment system unit, an RO system unit and an EDI system unit (namely above-mentioned one), three-level filtration is performed on raw water to obtain pure water in higher purity.

In an optional example, the pre-treatment unit includes: at least one filter, configured to filter sand grains, solid particles, colloid and microorganisms in the raw water, to obtain the first-level treated water.

In another optional example, the pre-treatment unit includes a pre-filter, a winding candle filter, a granular active carbon filter, and an ultrafiltration membrane element.

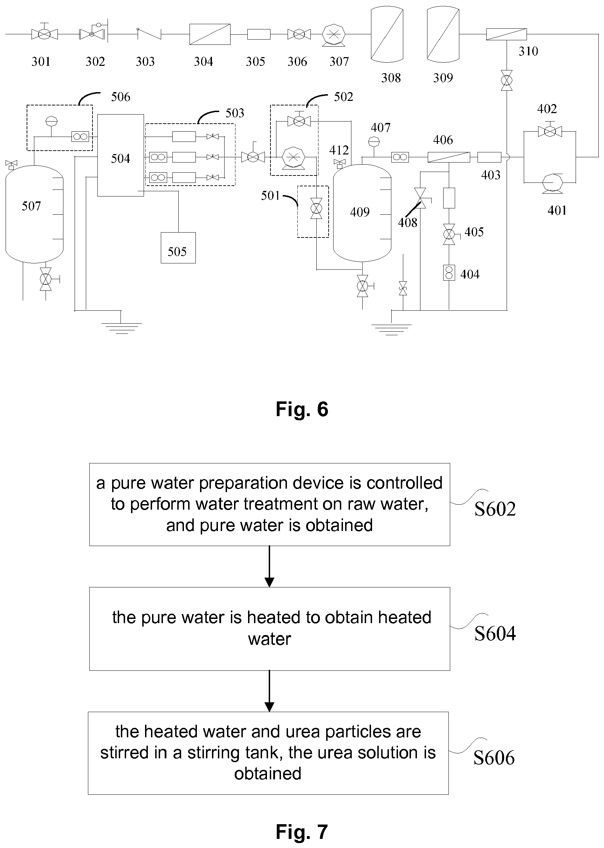

The above embodiment is described in detail in combination with FIG. 3 and FIG. 6, as shown in FIG. 3, the pure water preparation module may include: a manual ball valve 301, a reducing valve 302, a check valve 303, a pre-filter 304, a pressure sensor 305, an electric ball valve 306, a raw water boosting pump 307, a winding candle filter 308, a granular active carbon filter 309, and an ultrafiltration membrane element 310.

The pre-treatment unit includes a manual ball valve, a reducing valve, a check valve, an automatic washing stainless steel wire pre-filter in 40-um (namely the pre-filter in the above embodiment), a pressure sensor, an electric ball valve, a raw water pump, a stainless steel winding filter in 5-core 20-inch 10-um (namely the above winding candle filter), a stainless steel granular active carbon filter in 5-core 20-inch, a washable ultrafiltration membrane filter in 3t/hPVDF, and a washing electric ball valve.

It is to be noted that the connection relation between each filter and valve as shown in FIG. 3 may change the configuration according to a water quality condition of a device installation place, for example, the ultrafiltration membrane element may be eliminated in a region with good water quality, or an installation position of the ultrafiltration membrane element is forwards moved and the stainless steel filter and the winding candle filter are eliminated.

Through functions of the pre-treatment unit of removing sand, particles, colloid and microorganisms in feed water, a follow-up RO system is protected.

Specifically, the raw water in the embodiments of the disclosure may be qualified municipal administration tap water or other centralized water supply in comply with a GB5749-2006 standard. The raw water enters the pre-treatment unit through the started manual ball valve, a pressure of the feed raw water is guaranteed not to be greater than 0.4 MPa through the action of the reducing valve, air is guaranteed not to enter a complete machine system from a water inlet through the action of the check valve, and a failure risk generated because the air enters the system is eliminated. The automatic washing stainless steel wire pre-filter in 40-um may filter sand solid particles of greater than 40 um in the raw water, and pollutants stacked on the surface of a filter screen are automatically washed at regular intervals. The pressure sensor and the electric ball valve are combined to form a low-pressure protection mechanism, while the pressure of feed water sensed by the pressure sensor is lower than 0.2 MPa of the pressure designed by the system, the electric ball valve is closed, and the system (namely the pre-treatment unit) is stopped. The raw water is pressurized by the boosting pump, and successively enters the stainless steel winding filter in 5-core 20-inch 10-um and the stainless steel granular active carbon filter in 5-core 20-inch 10-um, solid particles of greater than 10 um in water are filtered through the winding filter, the granular active carbon filter may adsorb residual chlorine and partial organic matters in the water, the water entering the RO unit is guaranteed not to contain the residual chlorine, and the residual chlorine with the strong oxidizing property may produce a degradation destructive effect to a functional layer on the surface of an RO membrane after contacting with the RO membrane. Particles, colloid, partial organic matters, viruses and microorganisms of greater than 0.1 um may be removed from water after the filtration of the PVDF washable ultrafiltration membrane filter, a washing function to the membrane surface is realized by controlling the washing electric ball valve, while the flux of the PVDF ultrafiltration membrane is attenuated to 1 t/h which cannot be recovered by the automatic washing, and the ultrafiltration membrane filter element needs to be detached and the manual washing is performed.

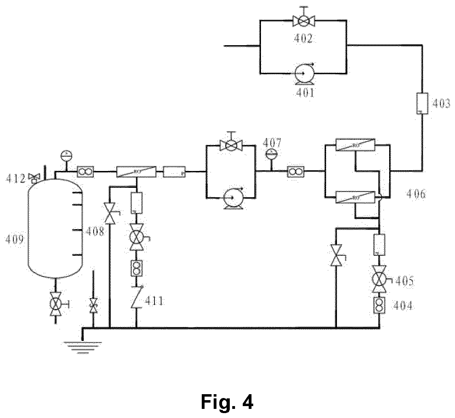

The RO treatment in the above embodiment is described in detail in combination with FIG. 4 and FIG. 6, as shown in FIG. 4, the RO treatment may include a two-level RO system unit, the two-level RO system unit mainly includes a RO high-pressure centrifugal pump 401, a manual ball valve 402, a pressure sensor 403, a pulse-type flowmeter 404, a stainless steel manual needle valve 405, an RO membrane component 406, a conductivity meter 407, a washing magnetic valve 408, a water generating tank 409, a water level control device, a check valve 411, and a respirator 412.

It is to be noted that the position relation of the RO unit as shown in FIG. 4 is an example illustration, the position relation of each part in the unit may be changed according to a water quality condition, optionally, the RO unit may be a two-level RO mode formed by three RO membrane components, or a first-level RO mode formed by one or more membrane components.

Pre-treated raw water (namely the first-level treated water) removes most of ions contained in the water under the action of the two-level RO treatment unit, and reaches the water feed requirement of an EDI unit.

Specifically, the pre-treated water enters a first-level RO high-pressure centrifugal pump and is pressurized to a working pressure (0.8 or 1.6 MPa) of a first-level RO component, the flow and the pressure may be adjusted within a certain range through a manual ball valve on a backflow branch parallelly connected with a pump, the pressure sensor and the pulse-type flowmeter may monitor the pressure and flow of output water of the high-pressure pump on line, while the flow is less than 1 t/h, the system sends out an alarm. The water pressurized by the high-pressure pump enters two first-level RO components for purifying, according to different water quality conditions in various regions, a pure water/concentrated water recovering rate may be adjusted through the stainless steel manual needle valve, a first-level RO recovering rate is generally set as 30%-50%, the pressure and flow of the discharged concentrated water are detected on line through the pressure sensor and the pulse-type flowmeter behind the stainless steel needle valve, in the case of setting once every operation/continuous operation for 1 hour/forced washing, two RO components are washed through the washing magnetic valve, the water generating pressure and the water generating flow of the first-level RO are detected on line through the pressure sensor and the pulse-type flowmeter, and the generated water quality is monitored on line through the conductivity meter, while the generated water quality is greater than 40 uS.cm, the system sends out an alarm.

The first-level RO generated water enters a two-level high-pressure centrifugal pump and is pressurized to a working pressure (0.8 or 1.6 MPa) of a two-level RO component, the flow and the pressure may be adjusted within a certain range through a manual ball valve on a backflow branch parallelly connected with a pump, the water pressurized by the high-pressure pump enters the two-level RO component and is purified, a pure water/concentrated water recovering rate may be adjusted through the stainless steel manual needle valve, a two-level RO recovering rate is generally set as 40%-90%, the pressure and flow of the discharged concentrated water are detected on line through the pressure sensor and the pulse-type flowmeter behind the stainless steel needle valve, in the case of setting once every operation/continuous operation for 1 hour/forced washing, the RO component is washed through the washing magnetic valve, the water generating pressure and the water generating flow of the two-level RO are detected on line through the pressure sensor and the pulse-type flowmeter, and the generated water quality is monitored on line through the conductivity meter, while the generated water quality is greater than 20 uS.cm, the system sends out an alarm. Qualified generated water is discharged to a middle water tank for collecting, the water tank is internally provided with a liquid level control device, the water tank is strictly sealed, and an air exhaust valve on the top of the water tank is used for exhausting a redundant gas and guaranteeing that external solid particles cannot enter the water tank.

Ultrafiltration washing waste water and concentrated water discharged by the two-level RO system, and washing water are discharged by a drainage main pipe in 1-inch, a concentrated water pipe of two-level RO is provided with the check valve for preventing the first-level concentrated water from affecting the discharge of the second-level concentrated water. The highest position of a drain pipe is provided with the air exhaust valve for exhausting the gas accumulated in the pipe, and the generation of a backpressure is prevented.

A working process of the first-level RO treatment unit in the above embodiment is the same as a first-level working process in the two-level RO system, while the generated water quality is greater than 20 uS.cm, the system sends out an alarm.

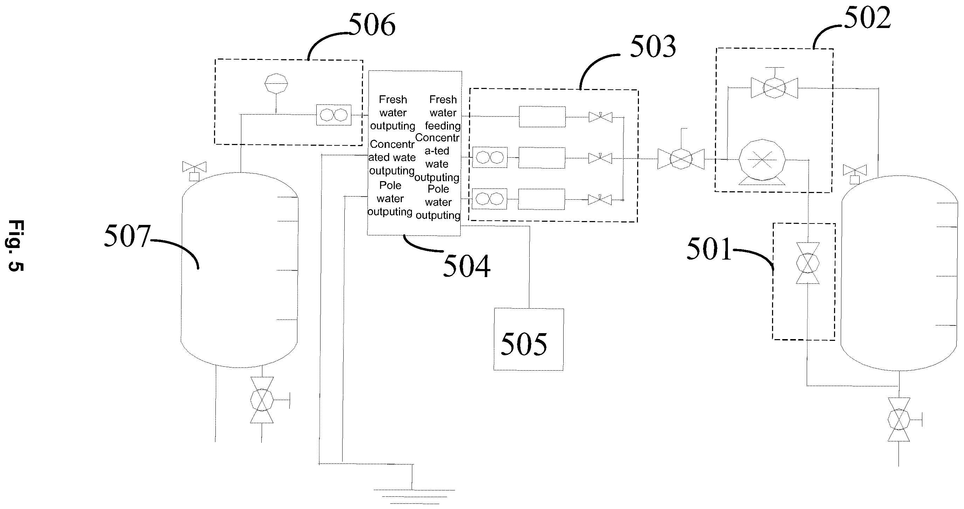

The above embodiment is described in detail in combination with FIG. 5 and FIG. 6 below, the pure water manufacturing device may be an EDI system (a system for manufacturing pure water by using a pure water manufacturing technology combined with an ion exchange technology, an ion exchange membrane technology and an ion electromigration technology), as shown in FIG. 5, the pure water manufacturing device includes: a feed water electric ball valve 501, a feed water pressurizing system 502, an EDI feed water detection adjusting system 503, an EDI module 504 and a power source 505, an EDI generating water online monitoring system 506 and a generating water collecting system 507.

Specifically, while the liquid level in a middle water tank reaches a designed height, the electric ball valve of the EDI system is started, the two-level RO generating water in the middle water tank is pressurized to about 0.3 MPa by the started boosting pump, the flow and the pressure may be adjusted within a certain range through a manual ball valve on a backflow branch parallelly connected with a pump, and the water enters an EDI feed water detection adjusting system, the EDI feed water is divided to three branches of fresh water, concentrated water and pole water, the fresh water flow is controlled within 80-120 L/h, the concentrated water flow is controlled within 12-18 L/h, and the pole water flow is controlled within 3 L/h, an operating voltage range is 50-100V, and a current is 0.2-0.3 A, specifically adjusted according to a practical condition, each water outlet of the module keeps the smooth water flow, and any backpressure is not existent. The pure water flow and the water quality are monitored on line through the pulse-type flowmeter and a resistivity meter, while a resistivity is lower than 0.2 MΩ·cm, the system sends out an alarm. The qualified generating water enters a strictly sealed product water tank with liquid level control through the electric ball valve, the upper part of the product water tank is provided with a respirator, the internal pressure balance is realized through the respirator, and external matters are guaranteed not to enter the water tank.

Optionally, the pure water preparation device further includes a detector, configured to detect content of carbon dioxide in the water in which the trace ions are filtered; and a degasser, configured to, in the case that the content of the carbon dioxide exceeds a preset content, remove the carbon dioxide in the water in which the trace ions are filtered.

In the case that the carbon dioxide in the water seriously affects the generating water quality, the degasser may be additionally installed for removing the carbon dioxide so as to guarantee the generating water quality, the degasser mainly includes a degassing membrane component and a vacuum pump.

Optionally, the pure water preparation device further includes: a resin component, configured to, after the EDI system is used for filtering trace ions in the second-level treated water, purify the water in which the trace ions are filtered.

In the case that the higher generating water quality (for example, 18 MΩ·cm) is required, a resin unit (for example, a polishing resin unit) is additionally installed behind the EDI component, the purification is further performed, compared with a common technology, the volume of the additionally installed polishing resin unit is greatly reduced.

The trace ions contained in the water may be further removed through an EDI treatment system, so that the water reaches a clean degree of pure water.

The stirring tank in the above embodiment is provided with a circulating pump, the circulating pump is configured to pump out the urea solution in the stirring tank from a circulating stirring output pipe, and enter from a circulating stirring input pipe, so that the circulating stirring is performed, wherein a connecting port of the circulating stirring input pipe and the stirring tank is positioned between the bottom of the stirring tank and the middle of the stirring tank.

According to the embodiment of the disclosure, an embodiment of a preparation method for vehicle urea solution is provided, it is to be noted that the steps as shown in flow diagrams in drawings may be executed in a computer system, such as a group of computer executable instructions, in addition, although a logic sequence is shown in the flow diagrams, in some cases, the shown or described steps may be executed in sequences different from the steps herein.

FIG. 7 is a preparation method for vehicle urea solution according to an embodiment of the disclosure, as shown in FIG. 7, the method includes the following steps.

Step S602, a pure water preparation device is controlled to perform water treatment on raw water, and pure water is obtained.

Step S604, the pure water is heated to obtain heated water.

Step S606, the heated water and urea particles are stirred in a stirring tank, the urea solution is obtained.

The preparation device for the vehicle urea solution in the above embodiment is a complete machine device, through the pure water preparation device, the heater, and the stirring tank, devices of pure water preparation, pure water heating, solution stirring and the like are integrated, each device is synergistically operated, the pure water preparation device is controlled to perform the water treatment on raw water, the pure water is obtained, the obtained pure water is stored in a pure water tank, the pure water stored in the pure water tank is heated to obtain heated water, the heated water and urea particles are stirred in the stirring tank, the urea solution is obtained, and the urea solution is pumped out, and stored in the storage box.

The preparation for the urea solution may be rapidly completed. In addition, the preparation device in the above embodiment is capable of breaking the original industrialized production mode, realizing the miniaturization and automation of the devices. Through the above embodiment, the problem in the related art that the low preparation efficiency of the urea solution is solved, and the preparation efficiency of the urea solution is improved.

According to the above embodiment of the disclosure, the step of controlling a pure water preparation device to perform water treatment on raw water includes the following steps: the raw water is pre-treated through a pre-treatment unit, and first-level treated water is obtained; the first-level treated water is filtered by an RO treatment unit, and second-level treated water is obtained; the second-level treated water is filtered by using an ion exchange technology, and the pure water is obtained.

Optionally, the step of pre-treating the raw water through a pre-treatment unit includes the following step: at least one filter is used for filtering sand grains, solid particles, colloid and microorganisms in the raw water, and the first-level treated water is obtained, wherein the pre-treatment unit includes at least one filter, namely the pre-treatment unit may be formed by at least one filter, or may be formed by at least one filter and other devices for realizing other functions.

More optionally, the step of pre-treating the raw water through a pre-treatment unit includes the following steps: a pre-filter, a winding candle filter, a granular active carbon filter, and an ultrafiltration membrane element are successively used for filtering the raw water, wherein the pre-treatment unit includes the pre-filter, the winding candle filter, the granular active carbon filter, and the ultrafiltration membrane element.

The step of filtering the first-level treated water by an RO treatment unit includes the following step: ions in the first-level treated water are removed by a two-level RO treatment unit, and the second-level treated water is obtained.

According to the above embodiment of the disclosure, the step of filtering the second-level treated water by using an ion exchange technology includes the following step: an EDI system is used for filtering trace ions in the second-level treated water.

Optionally, after the EDI system is used for filtering trace ions in the second-level treated water, the content of carbon dioxide in the water in which the trace ions are filtered is detected; in the case that the content of the carbon dioxide exceeds a preset content, a degasser is used for removing the carbon dioxide in the water in which the trace ions are filtered.

More optionally, after the EDI system is used for filtering trace ions in the second-level treated water, the method further including: a resin component is used for purifying water in which the trace ions are filtered.

Optionally, the step of heating the pure water includes the following steps: while the pure water is flowed into a heat-insulating water tank, a compressor is started, and under the drive of the compressor, heat is transmitted to a condenser, the heat is released to the water in the heat-insulating water tank, so that the pure water is heated.

Specifically, the step of starting a compressor includes the following steps: the temperature of the heat-insulating water tank is detected, in the case that the temperature of the heat-insulating water tank is lower than a first threshold value, the compressor is started; after the compressor is started, the method further including: in the case that the detected temperature of the heat-insulating water tank is higher than a second threshold valve, the compressor is controlled to be stopped.

While the pure water flowed into the heat-insulating water tank, a heating module is operated to heat according to a preset temperature, in the above embodiment, a heat pump principle is used, and non-contact heating is used for realizing a purpose of rapidly heating.

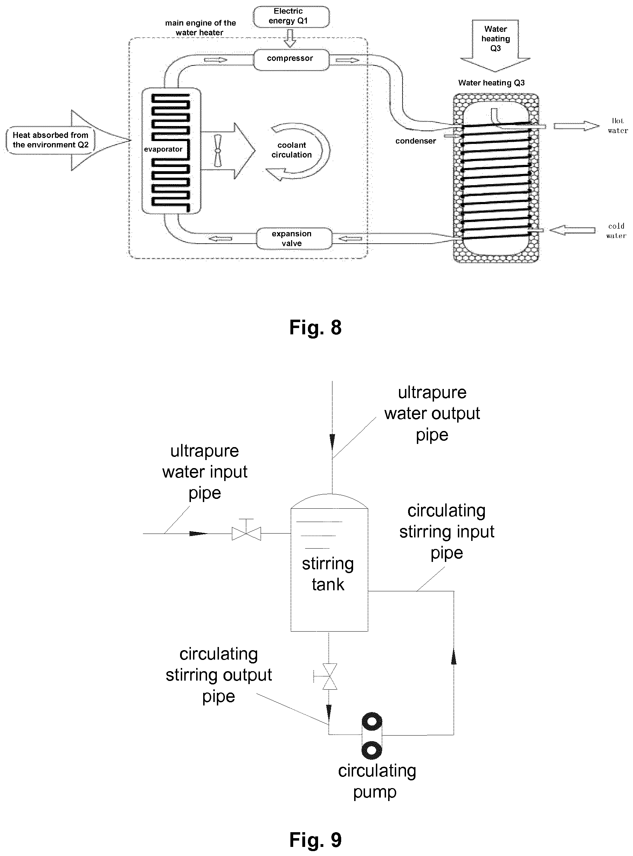

As shown in FIG. 8, a water heater mainly uses the heat pump principle, so that a part of electric energy is consumed as compensation, through a thermodynamic cycle, the heat is absorbed from a low-grade energy source (an air source) of a surrounding environment, the heat is transmitted to a condenser through a compressor, and released to water in a water tank, and the water is heated. A machine unit sets a shut-down temperature as 50 DEG C, while the machine unit detects that the temperature of the pure water tank is lower than 45 DEG C, the machine unit starts the heating, the pure water is heated, while the temperature of the water tank reaches 50 DEG C, the machine unit is shut down.

According to the above embodiment of the disclosure, the step of stirring the heated water and urea particles in a stirring tank includes the following steps: while a volume of the heated water entering the stirring tank reaches a first preset quantity, the stirring is started, and in the case that a preset condition is met, the urea particles are absorbed into the stirring tank; while the urea particles entering the stirring tank reach a second preset quantity, the urea particles are stopped to be absorbed into the stirring tank.

Optionally, the preset condition may be a condition that the heated water is adequately stirred, for example, the heated ultrapure water is completely stirred. Through the above embodiment, the heated ultrapure water is firstly added in a stirring process, and the circulating pump is started to stir the ultrapure water, after the ultrapure water is completely stirred, the urea particles are added to the stirring tank, so that the deposition of the urea particles is reduced and the stirring efficiency is improved.

In an optional embodiment, while the urea particles entering the stirring tank reach the second preset quantity, the feeding (for example, the urea particles) is stopped.

In an optional embodiment, after the stirring is started, the circulating pump is continuously controlled to circularly stir the heated water and the urea particles in the stirring tank, and the stirring operation is not interrupted or stopped because of feeding or stopping feeding.

Optionally, the step of circularly stirring the heated water and urea particles in the stirring tank includes the following steps: a circulating pump is used for pumping out solution in the stirring tank from a circulating stirring output pipe, and entering from a circulating stirring input pipe, so that the circulating stirring is performed until the concentration of the urea solution in the stirring tank reaches a preset concentration.

The stirring tank is mainly used for stirring and mixing the heated pure water and the urea particles, as shown in FIG. 9, while the pure water entering the stirring tank reaches the preset quantity, feed water is stopped, after a circulating pump is started to adequately stir the heated water, the urea particles are absorbed into the stirring tank, and the feeding is stopped while the particles reach the preset quantity, the circulating pump pumps out solution in the stirring tank from a circulating stirring output pipe, and the solution enters from a circulating stirring input pipe, the continuous circulating stirring is performed, until the uniformly mixed solution reaches a preset concentration.

In order to achieve the optimal stirring effect, a connecting port of the circulating stirring input pipe and the stirring tank is positioned between the bottom of the stirring tank and the middle (1/2 of a place) of the stirring tank. The relation between a cross area and a solution flow of the circulating stirring input pipe may accord with the relation as shown in Table 1:

**TABLE 1**

| | | | | |
|---|---|---|---|---|
| Flow (m³/h) | 3-4 | 4-6 | 6-8 | 8-12 |
| Cross area (mm²) | 50-120 | 100-170 | 150-250 | 200-330 |

For convenient description, the above is only an implementation mode, whereas in a specific implementation process, the installing modes of each part may be modified, for example, simple adjustment of a circulating pump installing positon as shown in FIG. 9, the effect of the disclosure is not affected.

The embodiment of the disclosure further provides a storage medium, the storage medium is used for storing a program for executing the following functions: a pure water preparation device is controlled to perform water treatment on raw water, and pure water is obtained; the pure water is heated to obtain heated water; the heated water and urea particles are stirred in a stirring tank, the urea solution is obtained.

The embodiment of the disclosure further provides a processor, the processor is used for executing a program for executing the following functions: a pure water preparation device is controlled to perform water treatment on raw water, and pure water is obtained; the pure water is heated to obtain heated water; the heated water and urea particles are stirred in a stirring tank, the urea solution is obtained.

The serial numbers of the embodiments of the disclosure are only used for descriptions, and do not represent the advantages or disadvantages of the embodiments.

In the above embodiments of the disclosure, descriptions of each embodiment are emphasized respectively, and parts which are not elaborated in detail in a certain embodiment may refer to relevant descriptions of other embodiments.

In some embodiments provided by the disclosure, it will be appreciated that the disclosed technological content may be implemented in other modes, wherein the apparatus embodiment described above is only schematic. For example, division of the units may be division of logical functions, and there may be additional division modes during actual implementation. For example, a plurality of units or components may be combined or integrated to another system, or some features may be omitted or may not be executed. In addition, displayed or discussed mutual coupling or direct coupling or communication connection may be performed via some interfaces, and indirect coupling or communication connection between units or modules may be in an electrical form or other forms.

The units that are described as separate components may be or may not be physically separated, and the components displayed as units may be or may not be physical units. That is, the units or components may be located at one place or scattered on several units. Some or all of the units may be selected according to actual needs to implement the solutions in the embodiments of the disclosure.

In addition, all function units in each embodiment of the disclosure may be integrated into a processing unit, or exist as independent physical units, or two or more units may be integrated into one unit. The integrated units may be implemented by using hardware, or by using the form of software function units.

If the integrated unit is realized in a mode of a software function unit and sold or used as an independent product, the integrated unit may be stored in a computer readable storage medium. Based on such understanding, the technical scheme of the disclosure or the part contributed to the existing technology or all or part of the technical scheme may be essentially reflected in a mode of a software product, the computer software product is stored in a storage medium which includes: a plurality of instructions is used for enabling a computer device (may be a personal computer, a server or a network device and the like) to execute all or partial steps of the method in each embodiment of the disclosure. The above-mentioned storage medium includes: various mediums for storing program codes, such as a U disk, a read-only memory (ROM), a random access memory (RAM), a mobile hard disk drive, a magnetic disk or a light disk.

The above are only preferred embodiments of the disclosure. It should be pointed out that those of ordinary skill in the art may also make some improvements and modifications without departing from the principle of the disclosure. These improvements and modifications should fall within the scope of protection of the disclosure.

### Industrial applicability

The technical scheme provided by the embodiments of the disclosure may be applied to a preparation process of vehicle urea solution, the preparation device for the vehicle urea solution is a complete machine device, through the pure water preparation device, the pure water tank, the heater, the stirring tank and the storage box, devices of pure water preparation, pure water heating, solution stirring and the like are integrated, each device is synergistically operated, so that the preparation for the urea solution may be rapidly completed. In addition, the preparation device in the above embodiment is capable of breaking the original industrialized production mode, realizing the miniaturization and automation of the devices. Through the above embodiment, the problem in the related art that the low preparation efficiency of the urea solution is solved, and the preparation efficiency of the urea solution is improved.

## Claims

1. A preparation method for vehicle urea solution, comprising:
controlling a pure water preparation device to perform water treatment on raw water to obtain pure water;
heating the pure water to obtain heated water; and
stirring the heated water and urea particles in a stirring tank to obtain the urea solution.

2. The method as claimed in claim 1, wherein a pure water preparation device is controlled to perform water treatment on raw water, comprising:
pre-treating the raw water through a pre-treatment unit to obtain first-level treated water;
filtering the first-level treated water a Reverse Osmosis, RO, treatment unit to obtain second-level treated water; and
filtering the second-level treated water by using an ion exchange technology to obtain the pure water.

3. The method as claimed in claim 2, wherein the raw water is pre-treated through a pre-treatment unit, comprising:
using at least one filter in the pre-treatment unit for filtering sand grains, solid particles, colloid and microorganisms in the raw water to obtain the first-level treated water.

4. The method as claimed in claim 2, wherein the pre-treatment unit includes a pre-filter, a winding candle filter, a granular active carbon filter, and an ultrafiltration membrane element; the raw water is pre-treated through a pre-treatment unit, comprising:
filtering the raw water by using the pre-filter, the winding candle filter, the granular active carbon filter, and the ultrafiltration membrane element in sequence.

5. The method as claimed in claim 2, wherein the first-level treated water is filtered by an RO treatment unit, comprising:
removing ions from the first-level treated water by a two-level RO treatment unit to obtain the second-level treated water.

6. The method as claimed in claim 2, wherein the second-level treated water is filtered by using an ion exchange technology, comprising:
filtering trace ions in the second-level treated water by utilizing an electrodeionization, EDI, system.

7. The method as claimed in claim 6, wherein, after the trace ions in the second-level treated water are filtered by utilizing filtering trace ions, further comprising:
detecting a content of carbon dioxide in water in which the trace ions are filtered; and
using a degasser for removing the carbon dioxide in the water in which the trace ions are filtered in a case that the content of the carbon dioxide exceeds a preset content.

8. The method as claimed in claim 6, wherein, after the trace ions in the second-level treated water are filtered by using EDI system, further comprising:
using a resin component for purifying water in which the trace ions are filtered.

9. The method as claimed in claim 1, wherein the step of heating the pure water to obtain heated water comprises:
starting a compressor while the pure water is flowed into a heat-insulating water tank, and transmitting heat to a condenser under the drive of the compressor, the heat is released to the water in the heat-insulating water tank, so that the pure water is heated.

10. The method as claimed in claim 9, wherein
The step of starting the compressor comprises: detecting the temperature of the heat-insulating water tank, in the case that the temperature of the heat-insulating water tank is lower than a first threshold value, starting the compressor; and
after the compressor is started, further comprising: controlling the compressor to be stopped in the case that the detected temperature of the heat-insulating water tank is higher than a second threshold valve.

11. The method as claimed in claim 1, wherein the step of stirring the heated water and urea particles in a stirring tank to obtain the urea solution comprises:
starting the stirring while a volume of the heated water entering the stirring tank reaches a first preset quantity, and absorbing the urea particles into the stirring tank in the case that a preset condition is met; and
stopping the urea particles to be absorbed into the stirring tank while the urea particles entering the stirring tank reach a second preset quantity.

12. The method as claimed in claim 11, wherein the heated water and urea particles are stirred in the stirring tank, comprising:
using a circulating pump for pumping out solution in the stirring tank from a circulating stirring output pipe, and entering from a circulating stirring input pipe, so that the circulating stirring is performed until the concentration of the urea solution in the stirring tank reaches a preset concentration.

13. The method as claimed in claim 1, wherein the method further comprising: storing the pure water in an ultrapure water tank after the pure water is obtained.

14. A preparation device for vehicle urea solution, comprising:
a pure water preparation device, configured to perform water treatment on raw water to obtain pure water;
a heater, configured to heat the pure water to obtain heated water; and
a stirring tank, configured to stir the heated water and urea particles to obtain urea solution.

15. The device as claimed in claim 14, wherein the pure water preparation device comprises:
a pre-treatment unit, configured to perform the pre-treatment on raw water to obtain first-level treated water;
an Reverse Osmosis, RO, treatment unit, configured to filter the first-level treated water, to obtain second-level treated water; and
a pure water manufacturing device, configured to filter the second-level treated water by using an ion exchange technology to obtain the pure water.

16. The device as claimed in claim 15, wherein the pre-treatment unit comprises:
at least one filter, configured to filter sand grains, solid particles, colloid and microorganisms in the raw water to obtain the first-level treated water.

17. The device as claimed in claim 15, wherein the pre-treatment unit comprises: a pre-filter, a winding candle filter, a granular active carbon filter, and an ultrafiltration membrane element.

18. The device as claimed in claim 15, wherein the pure water preparation device further comprises:
a detector, configured to detect content of carbon dioxide in the water in which the trace ions are filtered; and
a degasser, configured to, in the case that the content of the carbon dioxide exceeds a preset content, remove the carbon dioxide in the water in which the trace ions are filtered.

19. The device as claimed in claim 15, wherein the pure water preparation device further comprises:
a resin component, configured to, after trace ions in the second-level treated water are filtered by utilizing an electrodeionization, EDI, system, purify the water in which the trace ions are filtered.

20. The device as claimed in claim 14, wherein the stirring tank is provided with a circulating pump, the circulating pump is configured to pump out the urea solution in the stirring tank from a circulating stirring output pipe, and enter from a circulating stirring input pipe, so that the circulating stirring is performed, wherein a connecting port of the circulating stirring input pipe and the stirring tank is positioned between the bottom of the stirring tank and the middle of the stirring tank.

21. The device as claimed in claim 14, wherein the preparation device further comprises: an ultrapure water tank, the ultrapure water tank is configured to store the pure water.
